# EUROPEAN PATENT APPLICATION

(11) **EP 4 733 378 A1**
(43) Date of publication of application: **29.04.2026**
(21) Application number: 24831109.4
(22) Date of filing: 09.05.2024
(51) Int. Cl.: C12M 1/00, C12N 5/07, B01L 3/00

(54) **SYSTEM FOR SELECTING SPERMATOZOA**

(30) Priority: 26.06.2023 ES 202331134 U; 03.05.2024 WO PCT/ES2024/070269
(71) Applicant: Vitrolife Sweden AB, 400 92 Göteborg (SE)
(72) Inventor: RUIZ JORRO, Miguel, 46003 VALENCIA (ES); CALATAYUD LLISO, Carmen, 46003 VALENCIA (ES)
(74) Representative: D Young & Co LLP
(86) International application number: PCT/ES2024/070285
(87) International publication number: WO 2025/003534

(57) **Abstract**

The present invention relates to a system for selecting spermatozoa that comprises a dish having at least one recessed microfluidic circuit (6), where each microfluidic circuit (6) is formed by three cavities (1,2,3) and two channels (4,5). The two channels form a capital 'L' shape (L), with the length of the first channel (4) being greater than the length of the second channel (5). The first cavity (1) is the cavity in which the semen sample is deposited and said cavity is arranged at the free end of the first channel ( 4); the third cavity (3) is arranged at the free end of the second channel (5); and the second cavity (2) is arranged at the intersection between the first channel (4) and the second channel (5) and has a greater diameter than the first cavity (1) and the third cavity (3), the first channel (4) corresponding to a sector for dispersion and thermotaxis and the second channel (5) corresponding to a sector for sperm selection using rheotaxis.

## Description

### Technical field

The present invention relates to a microfluidic sperm selection system based on two channels with opposing flow currents, forming an angle between them, which can be integrated into an intracytoplasmic sperm injection (ICSI) dish, without the need for an external system to create these flows.

### Prior Art

Semen preparation and sperm selection are essential steps in assisted reproductive technology (ART) treatments, as any manipulation can have an impact on sperm viability, especially on the integrity of their genetic information, and this can influence reproductive success and the health of the newborn.

Sperm selection methods were developed with two fundamental objectives in mind: the removal of seminal plasma and the selection of the best sperm, essential steps in preserving their fertilization capacity and the correct development of the embryo.

The most commonly used sperm selection methods today are Wash and Swim-Up (WSU) and Density Gradient Centrifugation (DGC). In WSU, the semen sample, diluted in culture medium, is initially centrifuged to concentrate the cell fraction in a sediment, or pellet, and to separate it from the seminal plasma. Next, culture medium is carefully added to the pellet and the whole is incubated for a specific period of time to allow the motile sperm to migrate to the upper fraction. After a final wash of this fraction, the final sample is obtained, which will be used for *in vitro* fertilization or ICSI. The total duration of the process is 60 to 70 minutes. In DGC, the entire semen sample is placed in the upper part of two layers of density gradients of a colloidal medium prepared at different concentrations and centrifugation is performed. This produces a pellet containing the spermatozoa with the best motility. After an additional wash of this pellet by centrifugation, the final sample of motile sperm is obtained in approximately 40 minutes. Both methods have been widely proven to be effective in removing seminal plasma and selecting motile sperm, but they have a number of limitations. Firstly, centrifugation (WSU and DGC) and incubation temperature (WSU) generate oxygen free radicals (ROS, reactive oxygen species) that induce sperm DNA fragmentation (DNAF). This is relevant because of the relationship between DNAF and reproduction failure, particularly at the level of fertilization and embryonic development, especially in the case of couples with low-quality gametes. Secondly, both methods require the semen sample to be handled and transferred between containers (tubes or culture dishes) on numerous occasions, each of which represents a potential risk of confusion or loss of sperm. Thirdly, they require a long preparation time and also expensive equipment to carry out the entire process.

For all these reasons, one of the most important lines of research today focuses on the search for new methodologies that allow for safer sperm selection, reducing the iatrogenic impact on sperm related to both the generation of ROS and the number of steps required to prepare the sample. Also, it is important to achieve greater efficiency in the process by reducing time and costs.

The use of microfluidics has become a promising trend that meets these requirements. Currently, there are already several commercial devices designed to select motile sperm. All of them are external platforms, in the form of slides, most of them with predefined circuits engraved on the surface and, in many cases, using polydimethylsiloxane (PDMS). Several authors have described the usefulness of microfluidic systems for selecting motile sperm that also have intact DNA. The main difference between microfluidics and traditional sperm selection methods, such as WSU or DGC, is that centrifugation of the sample is not required for its processing. Also, they allow the final fraction of motile sperm to be obtained in approximately 20 minutes.

However, the application of microfluidics in ART, as currently understood, requires the use of devices that are not routinely used in assisted reproduction laboratories. This is a factor that adds complexity and cost to the process and does not completely eliminate the risk associated with sample handling, since transfer of sperm from the microfluidic device to the ICSI dish is still required. Also, there are cases, especially those with very severe dyspermia (very low sperm count, very low motility, or very few spermatozoa with normal morphology), in which it is possible that no sperm will reach the end of the microfluidic channel, so there is a risk of being left without any sample with which to perform ICSI, when the sperm are trapped in the solid-walled and solid-ceilinged tunnels of current microfluidic chips.

Moreover, it should be noted that not all microfluidic systems exploit sperm rheotaxis, as they do not have a current flow. The liquid remains static and it is the sperm that move, but not against a current.

In the search to improve the sperm selection process, several groups have proposed handmade microfluidic systems on the ICSI dish itself, based on the combination of drops of culture medium, so that a drop of the semen sample is placed at one point in the system and the sperm is subsequently selected at another point.

These systems have the advantage of simplicity and efficiency, but also several drawbacks, such as the manual factor when creating the system, which depends on the personal skill of the user and prevents absolute reproducibility of the system, and above all, the failure to exploit all sperm selection tools.

Most of the abovementioned systems do not have any current flow as they combine drops of identical volume. Others do combine two drops of different volumes, but as they have only one microfluidic channel, it is impossible to generate two opposing currents.

The present invention proposes a system that is just as easy or even easier to use, but much more sophisticated, as it takes advantage of the particular features demonstrated by the best sperm when they move, such as their tendency to swim toward the edges following a curvilinear rather than a rectilinear path, their ability to swim against a current due to the phenomenon of rheotaxis, and their tendency to swim from colder to warmer zones due to the phenomenon of thermotaxis. To exploit these characteristics, it is necessary to generate current flows and temperature gradients, but always taking into account the importance of keeping the characteristics of the ICSI dish intact, especially the temperature and pH of the drops in which the oocytes will be placed, so that the treatment is as effective as possible. Therefore, if the aim is to be able to integrate a microfluidic system with these characteristics into an ICSI dish, a fundamental parameter must be taken into account in order for the system to be truly viable in everyday clinical practice, and that parameter is the time it takes for the system to provide enough high-quality sperm to perform ICSI. The proposed system achieves this within between ten and fifteen minutes. This is less than half the time required by most commercially available systems. It is this particular feature that allows this system to be integrated into an ICSI dish.

There is an ICSI dish on the market which has a built-in microfluidic system (Fertdish, published in Lab on a Chip 21 (775) 2021). This system has a number of clear differences with the system that is the subject of the present invention. Firstly, there is no current flow in the course of travel and therefore no countercurrent system, so the sperm swim through the system but not against a current, meaning that the phenomenon of rheotaxis is not exploited to select the best sperm. Nor is there a temperature difference, so the phenomenon of thermotaxis is also not exploited to select sperm. Thirdly, the course of travel to be followed by the sperm is straight, so the possibility of selecting those with curvilinear progression, which may have more intact DNA than those with rectilinear progression, is not exploited. Also, the recommended culture medium for the microchannels is polyvinylpyrrolidone (PVP), a dense medium known to affect sperm movement, slowing their speed and reducing the tendency of the best sperm to swim toward the edges. The course of travel is covered by a solid material, so, unlike the proposed system, it is impossible to recover sperm if none of them reach the end point because they are trapped inside the system. Also, the system incorporates PDMS into the microinjection dish, the material with which the microfluidic system is constructed and is not commonly used to configure the ICSI dish. Finally, the system requires an incubation time of one to two hours at 37°C, which could affect the microenvironment of the drops intended to contain the oocytes.

WO 2022/184960 A1 also describes an ICSI dish that incorporates a sperm selection system based on the interaction of sperm with the oocyte's cumulus oophorus cells. It consists of a straight channel with a cavity in the center for placing the cumulus cells. There are two other cavities at both ends of the channel. The cavity at one end is for placing the sperm sample and the one at the other end is for collecting the sperm that have reached it and using them for ICSI. Said system does not select sperm by rheotaxis, as there is no deliberate current flow in the channels that causes the best spermatozoa to swim against the current and only these to reach the final drop. Selection is based on the interaction of the sperm with the cumulus oophorus cells. It has the following drawbacks:
- a different material, the cumulus oophorus cells, is incorporated into the ICSI dish than that commonly used to configure the ICSI dish;
- the oocytes must be manipulated to remove these cumulus cells;
- the patient's oocytes may have poor-quality cumulus cells;
- the collection of these cumulus cells and their placement in the sperm selection system is done manually, which reduces the reproducibility of the system and makes it more user-dependent;
- some sperm could swim along the edge of the channel without interacting with these cumulus cells;
- the dish must be incubated at 37°C, which could generate oxygen free radicals (ROS) that could affect the integrity of the sperm DNA;
- it requires a prolonged incubation time for the entire dish to select the sperm, possibly because there is no effect that causes them to swim to the end of the course of travel. Also, they have to do so through the cumulus cells, which can prolong recovery time. This can have several negative consequences on the pregnancy prognosis when performing ICSI in this system, as the drops that have been placed on the dish for ICSI will have been prepared for a long time by the time the oocytes are transferred thereto, and their pH may have changed, which could affect the correct functionality of said oocyte;
- if the channels are very deep or protrude from the bottom of the dish, it will be difficult to recover the sperm from the final drop with the ICSI pipette.

The present invention provides a microfluidic sperm selection system consisting of one or more microfluidic circuits having two channels with opposing flow currents, forming an angle between them, which can be integrated into an ICSI dish, the objectives of which are to improve the effectiveness of sperm selection, to simplify it, and to make it safer and more efficient. The system takes advantage of the natural characteristics of movement of the best sperm, reduces the manipulation of the gametes and thus the iatrogenic damage thereto, simplifies the selection process, making it faster and cheaper, thereby improving efficiency, and, in the preferred embodiment in which the system is integrated into the ICSI dish, it reduces the risk of confusion when identifying the sample by not having to transfer it from one container to another, since all that needs to be done is to place a drop of semen on the ICSI dish and wait to recover the sperm that reach the end of the course of travel, which will be selected for microinjection into the oocytes on the same dish.

To do this, the microfluidic systems on the dish are not static, but have a current flow, in fact, two opposing current flows that prevent seminal plasma, dead cells or lower-quality sperm from reaching the end of the course of travel. Also, the circuit layout is not straight, but rather both currents form a right angle to improve the selection of the best sperm and ensure that no unwanted elements can be carried to the end. It has the advantage of using the same elements, as regards culture media and consumables, that are commonly used when configuring the drops on an ICSI dish to perform the treatment, without introducing media, materials or cells that are foreign to this process, making it simpler. It also does not require incubating the semen sample at a high temperature, which reduces ROS production and thus damage to the sperm, and shortens the recovery time. By shortening the preparation time, the drops of culture medium that will contain the oocytes are not affected. In addition, the entire microfluidic circuit is minimally engraved into the surface of the dish and does not protrude therefrom, which, as it is not covered by any solid material, allows the sperm to be easily recovered from any point along the course of travel.

In this way, the system allows the following:
1. selection of the sperm to be microinjected on the ICSI dish itself, thus avoiding successive transfers of the sample, thereby reducing the risk of confusion when identifying it, and also reducing the risk of sample contamination;
2. selection of the sample from whole semen, i.e. without prior processing of the sample, reducing the risk of iatrogenic damage to the sperm produced, especially but not exclusively by centrifugation, thus improving the effectiveness of the treatment;
3. selection of the best sperm by exploiting their specific characteristics of swimming toward the edges, swimming against the current (rheotaxis), and swimming toward warmer zones (thermotaxis), thus improving the effectiveness of the treatment;
4. simplification of the sperm selection technique for ICSI, reducing the time needed to obtain sperm for microinjection and the cost of inventory and consumables, which together improves efficiency compared to conventional sperm selection techniques for ICSI;
5. treatment of the majority of semen samples, unlike the limitations of other microfluidic systems in samples with severe dyspermia;
6. possibility of recovering sperm that have not reached the end zone of the course of travel;
7. scaling of the system, allowing its use in different types of treatment other than ICSI (*in vitro* fertilization or artificial insemination).

### Explanation of the Invention

The invented system combines the use of physical and biological principles that allow the selection of the best sperm, such as:
1. the tendency of sperm to swim against a current (rheotaxis);
2. the tendency of sperm to swim toward the edges;
3. the tendency of sperm to swim toward warmer zones (thermotaxis);
4. Laplace's law;
5. laws of hydrostatics.

To improve the prognosis of ICSI assisted reproduction treatment, it is essential to select the best sperm, which are defined as those with the best potential to fertilize the egg and develop a healthy embryo that will evolve until birth. It is known that sperm with poorer morphology and, especially, those with DNA integrity defects are associated with lower ICSI fertilization rates, higher incidence of embryonic development arrest, poorer embryo implantation rates, higher miscarriage rates, and poorer live birth rates per embryo transfer. The morphology or motility of a spermatozoon does not indicate whether or not its DNA is intact.

It has been shown that the best sperm, those with the highest DNA integrity, tend to swim against the current. This phenomenon is known as rheotaxis. Conversely, this capacity is impaired in the case of spermatozoa with DNA defects, moving less or being carried away by the current. This tendency to swim against the current appears to be a passive effect. The best sperm beat their tails more and with greater amplitude, becoming a kind of virtual sail that causes the effect of the current on them to direct the head of the spermatozoa against that current. It has been shown that applying a current flow in a microfluidic system (systems with very small amounts of fluid in sub-centimeter dimensions) selects sperm with a higher probability of having intact DNA.

It has also been shown that the best sperm tend to swim toward the edges, not in a straight line, but moving progressively to one side or the other. It has been shown that when sperm are made to swim through a microfluidic channel with right and left turns in its path, those that continued to swim in a straight line to the end of the channel, without deviating due to the bends, had a higher incidence of DNA fragmentation than those that swam close to the wall of the channel and therefore exited through the bend. This improvement in the rate of DNA integrity is independent of sperm motility or morphology, since all the spermatozoa had good motility that allowed them to move progressively. It has also been observed that more spermatozoa with intact DNA are recovered in bends when they have a 45° angle than when the angle is 90°.

It has been shown that the best sperm tend to swim from colder to warmer zones. This phenomenon is known as thermotaxis, and exploiting it reduces sperm recovery time and also allows for the selection of those with greater DNA integrity.

It has been shown (Laplace's law) that drops of the same liquid but with different radii have different pressures, with the pressure being higher in those with a smaller radius. This means that when two drops with different radii are connected, a flow or current is created that goes from the drop with the smaller radius to the one with the larger radius. This principle allows the creation of a microfluidic system with a current flow without the need for an external system, such as a pressure pump, to create these flows.

It has also been demonstrated (laws of hydrostatics) that the pressure on a given point within a fluid is proportional to the distance from that point to the surface. Therefore, when one drop with a certain volume and another with a larger volume are placed on a Petri dish and both are covered with oil, the pressure of the oil on the surface of each drop will be different, being greater in the drop with the smaller volume, because there is more distance between its surface and the surface of the oil, than in the drop with the larger volume, whose surface will be closer to the surface of the oil. This concept can also be used to create a pressure difference between two drops in a microfluidic system and, thereby, a current flow that persists over time without the need for an external system, which would be unfeasible in the ICSI dish.

Thus, the sperm selection system that is the subject of the present invention consists of one or more microfluidic circuits (or microfluidic and rheotactic sperm selection circuits) engraved in a dish, in which controlled flows of opposing currents are created, without the need for any external system, by connecting drops with different pressures obtained by the specific dimensions of each drop, in a path with specific deviations that allow the best sperm to be selected. A specific temperature gradient is also added to speed up this sperm selection and make it even more effective.

Preferably, the sperm selection system that is the subject of the present invention is incorporated into a conventionally used polystyrene ICSI dish in which the microfluidic circuit has been engraved.

Although many microfluidic systems are constructed from polydimethylsiloxane (PDMS), this one is preferably constructed from polystyrene or similar material, as this is the material commonly used in this type of ICSI dish and is generally a preferred material for cell culture.

Optionally, the sperm selection system is located on one side of the dish, so that the rest of the dish can be used to perform ICSI in the conventional manner.

Alternatively, the sperm selection system that is the subject of the present invention consists of one or more microfluidic circuits engraved in a dish independent of the ICSI dish, in which dish sperm selection is performed for subsequent use thereof in a conventional ICSI dish.

In general, when the sperm selection system is incorporated into an ICSI dish, there will be three distinct zones: one zone is for sperm selection, in which the microfluidic systems and the drops to which the selected sperm will be transferred are located; one zone for microinjection, in which the drops containing the oocytes to be microinjected can be placed. This zone generally occupies the center of the dish, as it is the zone where the temperature can best be controlled; also, a third zone where drops of culture medium can be placed to wash the oocytes as they are transferred from one culture medium to another.

The same dish can include multiple microfluidic circuits so that sperm selection can be performed in more than one thereof in order to recover more sperm from a larger initial sample volume (for example four drops of semen instead of a single drop).

Each circuit consists of two sectors: a first sector for dispersing the semen sample and sperm selection by thermotaxis, and a second sector for sperm selection by rheotaxis.

The final appearance of the complete course of travel, seen from above, is that of a capital L, with a longer arm (first channel) and a shorter arm (second channel), in which there is a well at each end and a well at the intersection of both sides. Preferably, and without limitation, the cross section of the channels is rounded rather than square, so as to better aspirate the sperm with the ICSI pipette if necessary.

The long arm of the L would correspond to the dispersion and thermotaxis sector and the short arm to the sector for sperm selection by rheotaxis.

The length of each sector or arm of the L corresponds to the specific requirements of the circuit. In general, it is desirable to make the course of travel as short as possible, so that the spermatozoa have to swim less distance and can be selected more quickly. However, an excessively short distance in the first sector could cause seminal plasma and dead cells to reach the intermediate drop. In this first channel, the current flows from the first drop, where the semen is deposited, to the second, to disperse the clot of the ejaculate sample, encourage the spermatozoa to leave it, with the best ones swimming toward the edges, and passively bring them closer to the rheotaxis zone. However, it is possible that some will turn around and swim against the current. To ensure that the best spermatozoa continue to move from drop 1 to drop 2, a temperature gradient is established between them, so that the best sperm will tend to move toward the second drop.

There, those that continue swimming along the edge of the channel will encounter a countercurrent flow coming from the second channel. The best sperm, due to their rheotaxis, will continue swimming against the current toward the final third drop, while the lower-quality sperm will be retained in the second drop. It is therefore in this second channel that the selection of the best sperm takes place. This second course of travel can be shortened compared to the first, but not excessively, because if the drops were too close together, the channel connecting them would lose its appropriate morphology.

Each circuit is engraved into the bottom of the dish, and from above it looks effectively like an L formed by two perpendicular channels and three wells. The two wells at the ends (first and third wells, the first well being the well at the start of the course of travel and the third well being the well at the end of the course of travel) are preferably of the same size, while the well at the intersection (second well) is larger, preferably twice as large. The first channel is also longer than the second (preferably twice as long).

Optionally, the two channels and the three wells are of equal depth.

Preferably, the first channel has a progressive slope so that it is deeper at the start (at the end in contact with the first well) and shallower at the end (at the end in contact with the second well). This configuration facilitates the deposition of immotile cells at the bottom of the channel, while motile sperm can swim above this deposit of immotile cells.

Preferably, the first channel has a progressive widening in its final section, near the end in contact with the second well. This widening at the end of the first channel has two objectives. Firstly, the widening of the channel reduces the speed of the current, which makes it easier for the best sperm, which swim close to the edge of the channel, to continue doing so and be diverted into the second channel, since a faster current could carry them straight through and prevent them from entering that second channel. Secondly, it has been shown that this inverted funnel morphology produces a divergent flow, which means that the direction of the flow remains the same at the edges of the channel, but that a turbulence effect occurs in the central zone of the channel, which would hinder the progress of sperm that do not swim close to the edge of the channel and which are theoretically of poorer quality.

Preferably, the second channel has a progressive narrowing at its start, so that it is wider at its end in contact with the second well. This progressive narrowing at the start of the second channel produces the same divergent flow effect that facilitates the countercurrent advance of sperm that continue to swim along the edge of the drop and hinders the entry into the second channel of sperm that swim in the center. Also, the speed of the current will be greater at the end of this second channel, which is where the selection of the best sperm must take place.

Preferably, the intersections between the wells and the channels are curved, avoiding open angles. These smooth junctions between the channels and the wells help to maintain contact between the best sperm and the edge of the channel. If the zone where the channel meets the well forms a right angle, the sperm is more likely to separate from the edge, whereas a smooth intersection makes it more likely that the sperm will remain close to the edge of the microfluidic circuit.

Preferably, the well at the end of the course of travel has sperm recovery zones formed by progressively shallower protruding parts that cause the sperm to enter and remain trapped therein, preventing them from continuing to swim and returning backwards and instead remaining in these zones, ready to be aspirated and used for ICSI.

Optionally, along the second channel there is also a set of sperm recovery zones formed by progressively shallower protruding parts, arranged on one side of the channel or on both sides.

Preferably, the sperm selection system is incorporated into an ICSI dish and consists of at least two microfluidic systems in order to be able to perform the process in duplicate and at the same time be able to recover more sperm.

The sperm selection system is prepared by placing a drop of the same volume of culture medium in each well and then a fourth drop of the same volume on top of the drop in the middle well, so that this drop will have twice the volume of the others.

Depending on the finish of the plastic dish (hydrophobic or hydrophilic), the drops may connect together automatically as the channels fill by capillary action, or a sterile pipette tip may need to be passed over the channels, from one drop to another, to connect them. In any case, as soon as the three drops are connected, a pressure difference will automatically be created between them.

Once the microfluidic circuit has been primed with the culture medium, if the sperm selection system is incorporated into an ICSI dish, the rest of the ICSI dish is configured by placing the drops to which the sperm selected for ICSI and the oocytes (eggs) will later be transferred, and the whole is covered with mineral oil.

Once this is done, a drop of semen, half the volume of the previous ones, is added to the first well of the system, which is located at the end of the longest channel.

Next, a temperature gradient is generated between the first and second drops of the system, so that the first drop is colder. To achieve this, the dish incorporating the system (preferably an ICSI dish, although it can be a separate dish) is placed on a plate made of a material with high thermal conductivity (for example a 2 mm aluminum plate) that has a specific configuration with a series of cutouts, so that said plate is in contact with the entire dish except for the wells in which the semen sample will be placed.

Finally, the dish-plate assembly is placed on a heated flat surface. The aluminum will transfer the temperature to the entire dish so that the temperature of all the drops is preferably 37°C, except for the drops containing the semen sample, which will remain cooler. This creates a temperature gradient between the first and second drops, which is necessary for the thermotaxis process to occur.

Heating the system except for the drop in which the semen sample has been deposited has two purposes. Firstly, it reduces the temperature in the drop in which the whole semen is placed, since an increased temperature causes cells with more cytoplasm, such as white blood cells, germline cells, and immature spermatozoa, to generate oxygen free radicals (ROS) that can damage the DNA of healthy sperm. Secondly, it creates a temperature gradient between the first and second drops, which means that more sperm are recovered in less time due to the tendency of the best sperm to swim toward warmer zones as a result of thermotaxis.

Preferably, there is a cover to be able to cover the dish during the sperm selection process, which reduces the contact of mineral oil with the environment, thus promoting the stability of the dish conditions in terms of temperature, pH, and osmolarity, while preventing the risk of contamination.

According to the results, in most semen samples with at least one million spermatozoa with good rectilinear progression per milliliter, in less than fifteen minutes there will be enough sperm to microinject at least 20 oocytes in the final drop of the circuit, and in cases of normozoospermia (semen samples with all parameters within the WHO normal reference limits), in less than ten minutes.

The drops are of different diameters and volumes, so that, according to Laplace's Law, the pressure of each drop is inversely proportional to its diameter. Thus, a drop of a given diameter will have more pressure than a drop of the same culture medium with a larger diameter, and when the two drops are joined, a flow will be created, i.e. a displacement of the medium from the drop with more pressure to the drop with less pressure.

Also, as is customary in the ICSI technique, the drops are covered with mineral oil to prevent changes in pH and osmolarity in the culture medium due to evaporation. This oil covering the drops, in accordance with the laws of hydrostatics, also exerts a different pressure on the smaller-volume drops than on the larger-volume drops, so that this pressure is also inversely proportional to the volume of the drop.

Taken together, this means that when two drops of different sizes are joined by a channel of the same culture medium, this medium will gradually move from the smaller drop to the larger drop. Further, the smaller the small drop and the larger the large drop becomes, the greater the pressure difference between them will be, so that this flow will continue for some time.

This time and the speed of the flow can be regulated by modifying the volume and diameter of the drops, the distance between them, and the dimensions of the channel connecting them.

The system that is the subject of the present invention is also characterized in that it involves the following procedure:
- placing a drop of the same volume of culture medium in each well and then a fourth drop of the same volume on the drop in the middle well, so that it will have twice the volume of the others;
- passing a sterile pipette tip over the channels, from one drop to another, so that they become connected;
- in the preferred embodiment in which the system is incorporated into an ICSI dish, configuring the rest of the ICSI dish by placing thereon the drops that will contain the selected sperm and the oocytes (eggs);
- covering the entire dish with mineral oil;
- adding a drop of semen, half the volume of the previous ones, to the first well, which is at the end of the longest channel;
- placing the cover on the dish;
- placing the dish on a plate made of a material with high thermal conductivity;
- placing the assembly of dish and plate of material with high thermal conductivity on a flat surface heated to 37°C. The plate will transfer the temperature to the entire ICSI dish except for the drop containing the semen sample, creating a temperature gradient between the first and second drops, which is necessary for the thermotaxis process to occur;
- aspirating the sperm accumulated in the sperm recovery zones, preferably using an ICSI micropipette.

### Brief Description of the Drawings

To complement the description being given and in order to aid in better understanding of the characteristics of the invention, in accordance with a preferred example of its practical embodiment, a set of figures is included as an integral part of said description, in which, for illustrative and nonlimiting purposes, the following have been represented:
Figure 1 shows a schematic view of the microfluidic circuit according to one embodiment of the present invention.
Figure 2 shows a top view of a round ICSI dish with two microfluidic and rheotactic sperm selection circuits, according to one embodiment of the present invention.
Figure 3 shows a top view of a round ICSI dish with four microfluidic and rheotactic sperm selection circuits, according to one embodiment of the present invention.
Figure 4 shows a schematic of the possible overall dimensions of two sperm selection circuits, according to one embodiment of the present invention.
Figure 5 shows a cross section of the system shown in Figure 4.
Figure 6 shows a cross section of the system shown in Figure 4.
Figure 7 shows a schematic of the possible overall dimensions of two sperm selection circuits, according to one embodiment of the present invention.
Figure 8 shows a detailed view showing the rounded junctions between the channels and the wells.
Figure 9 shows an example configuration of the ICSI dish of Figure 2 with the plate made of a material with high thermal conductivity underneath it to achieve a temperature difference between the first and second drops of each system, according to one embodiment of the present invention.
Figure 10 shows a top view of a rectangular ICSI dish with a possible layout of two microfluidic and rheotactic sperm selection circuits, according to one embodiment of the present invention.
Figure 11 shows the same dish as in Figure 10 with a plate made of a material with high thermal conductivity underneath it to achieve a temperature difference between the first and second drops of each circuit.
Figure 12 shows a top view of a rectangular ICSI dish with another possible layout of two microfluidic and rheotactic sperm selection circuits, according to one option of the present invention.
Figure 13 shows the same dish as in Figure 12 with the system of a plate made of a material with high thermal conductivity underneath it to achieve a temperature difference between the first and second drops of each system.
Figure 14 shows an example of preferred locations for the sperm accumulation and recovery zones based on the relationship between the circuit layout and the direction of entry into the dish of the ICSI micropipette, according to one embodiment of the present invention.
Figure 15 shows an example of preferred locations for the sperm accumulation and recovery zones based on the relationship between the circuit layout and the direction of entry into the dish of the ICSI micropipette, according to one embodiment of the present invention.
Figure 16 shows an example of an embodiment of the invention in a dish independent of the ICSI dish comprising a set of engraved microfluidic circuits.
Figure 17 shows the dish of Figure 16 with the plate made of a material with high thermal conductivity underneath it to achieve a temperature difference between the first and second drops of each system, according to one embodiment of the present invention.

### Description of the Preferred Embodiments of the Invention

In the light of the abovementioned figures, and in accordance with the numbering adopted, it may be seen that they show an example of a preferred embodiment of the invention, which comprises the parts and elements indicated and described in detail below.

Thus, the system that is the subject of the present invention comprises an ICSI dish (7) that has at least one microfluidic circuit (6) engraved therein.

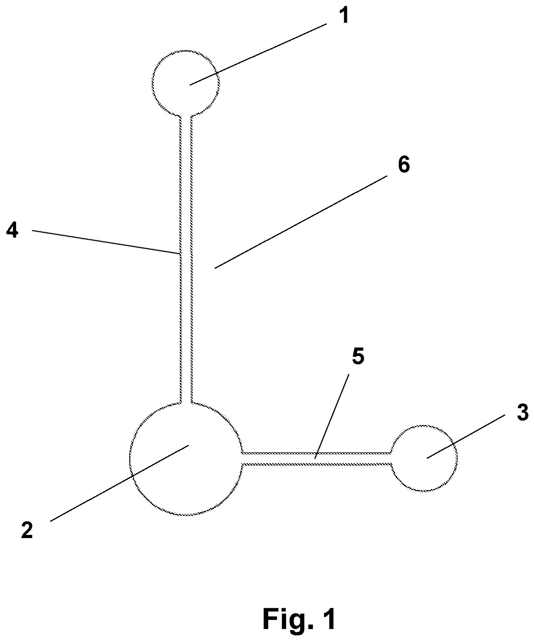

As shown in Figure 1, the microfluidic circuit (6) consists of three wells (1, 2, 3) and two channels (4, 5), in which the two channels form a capital L, with the length of the first channel (4) being twice the length of the second channel (5). The first well (1) is the well in which the semen sample is deposited and is located at the free end of the first channel (4). The third well (3) is the well from which the selected spermatozoa are recovered, and is located at the free end of the second channel (5), and has the same diameter as the first well. The second well (2) is located at the intersection between the first channel (4) and the second channel (5) and has twice the diameter of the first well (1) and the third well (3), with the first channel (4) corresponding to a dispersion and thermotactic sector and the second channel (5) to a rheotactic sperm selection sector.

Thus, the system is formed by joining three wells of different volumes, so that the second well (2) has twice the volume of the first and third wells (1 and 3) so that, according to Laplace's law and the laws of hydrostatics, the first and third wells have more pressure than the second well when they contain fluid inside. By connecting the wells via the channels (4 and 5), a current flow is created from the first and third wells at the ends (1, 3), which have more pressure, toward the central second well (2).

When a semen sample (6) is placed in the first well (1), pressure exerted toward the second well (2) causes the seminal plasma to disperse through the first channel (4) and the spermatozoa with the best curvilinear motility to move toward the edges of the channels and advance with the flow. When they reach the second well (2), they encounter a current flowing from the third well (3). The best spermatozoa will continue to swim along the edges and manage to overcome the countercurrent and reach the third well (3). To prevent them from continuing to swim backwards, so that they instead accumulate in the third well (3) as they arrive, it has small, progressively shallower protruding parts that cause the spermatozoa to enter and remain trapped therein, ready to be aspirated and used for ICSI.

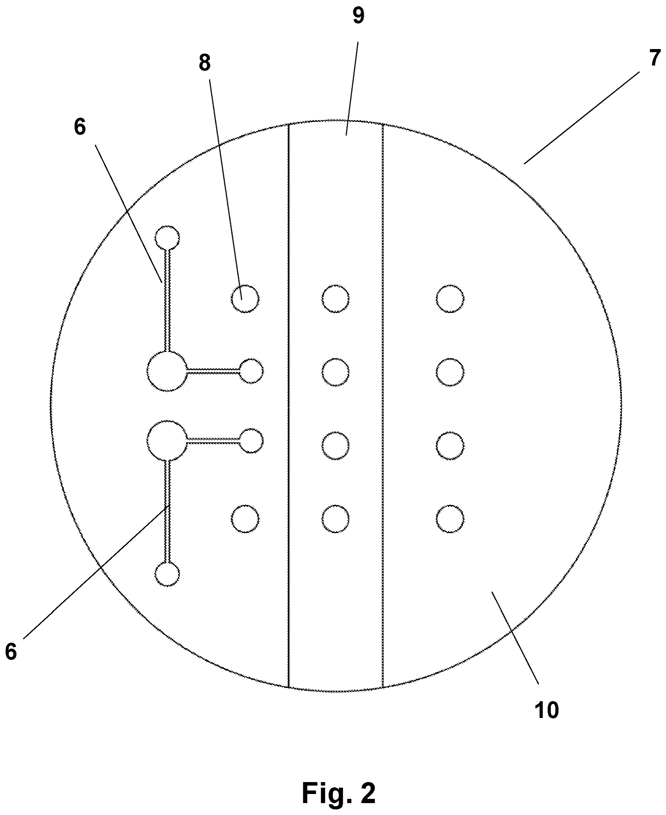

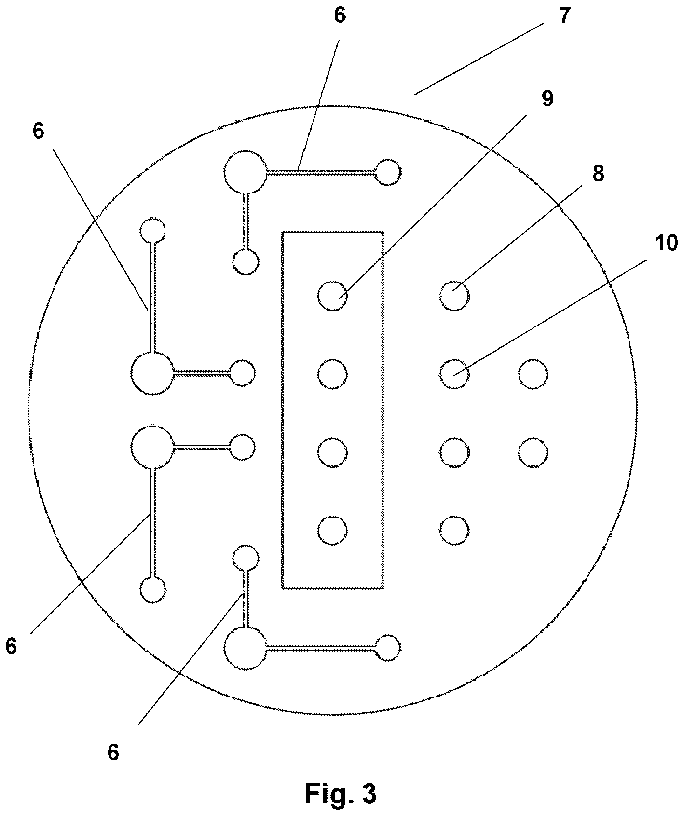

The right angle formed between the channels and the opposing current flow in each of them prevents seminal plasma, immotile cells, or low-quality spermatozoa from reaching the third drop. Only the best spermatozoa will be able to swim against the current and reach the final drop. Figures 2 and 3 show two examples of ICSI dishes (7) with two and four microfluidic and rheotactic sperm selection systems.

The rest of the dish is left free to freely arrange the remaining drops in that zone to carry out the ICSI process. The examples shown in Figures 2 and 3 indicate a possible sperm selection zone (18) containing: engraved microfluidic circuits (6) and other zones (8) for optionally placing drops of polyvinylpyrrolidone (PVP) or another dense medium where the spermatozoa aspirated from the recovery zones are placed to be immobilized and subsequently microinjected; a washing zone (10) with zones for placing drops of culture medium where the oocytes can be progressively washed; a microinjection zone (9) with zones for placing drops of culture medium in which the oocytes will be placed for microinjection.

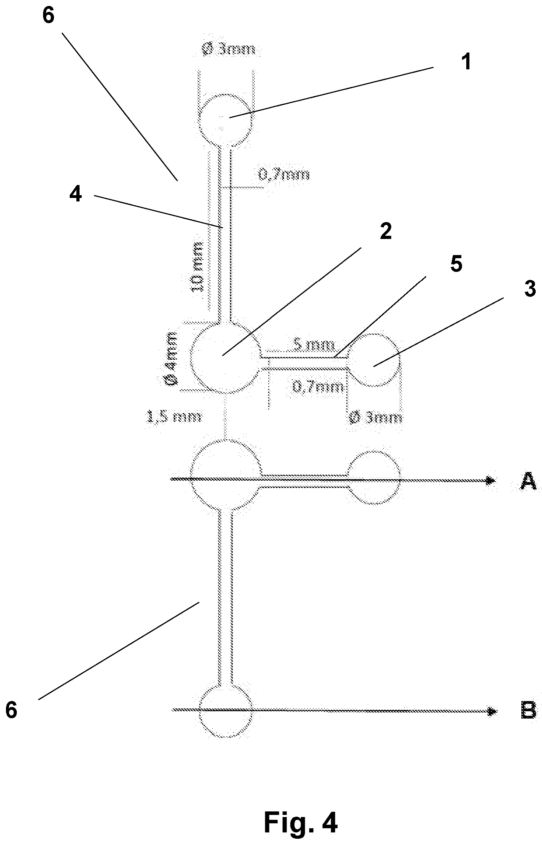

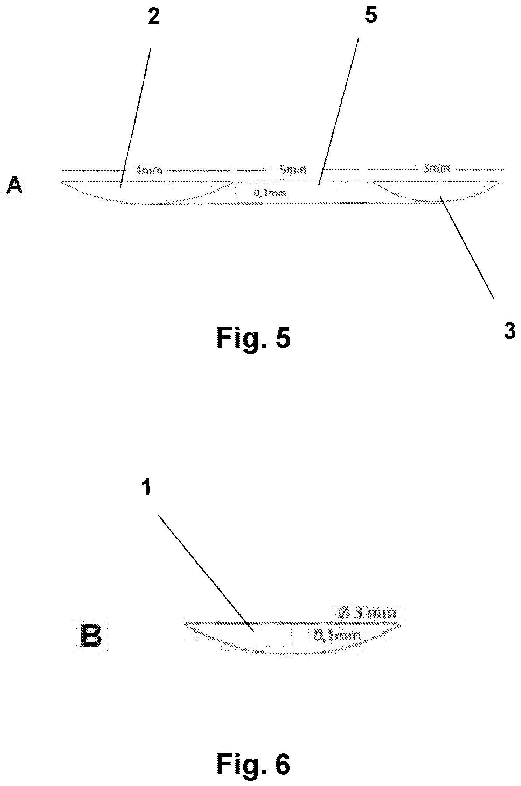

Figures 4 and 5 show a possible embodiment example of the microfluidic circuits (6). In the embodiment shown, the first well (1) and the third well (3) have a diameter of 3 mm and a depth of 0.1 mm, while the second well (2) has a diameter of 4 mm and a depth of 0.1 mm. The first channel (4) has a length of 10 mm and a width of 0.7 mm, while the second channel (5) has a length of 5 mm and a width of 0.7 mm.

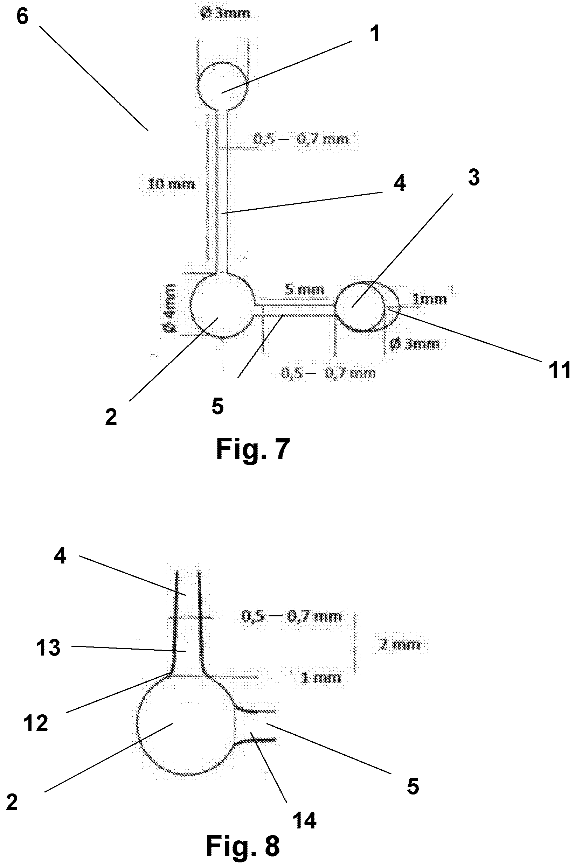

Figures 7 and 8 show another possible embodiment example of the microfluidic circuits. The width of both the first channel (4) and the second channel (5) is in the range from 0.5 to 0.7 mm. The first channel (4) has a section of increasing width (13) in the last 2 mm of its length near the second well (2), increasing in width from 0.5-0.7 mm to 1 mm. The second channel (5) has a section of decreasing width (14) in the first 2 mm of its length near the second well (2), decreasing in width from 1 mm to 0.5-0.7 mm. The circuit also comprises a sperm accumulation and recovery zone (11) of progressively reduced depth, from 0.1 mm to 0 mm over a distance of 1 mm.

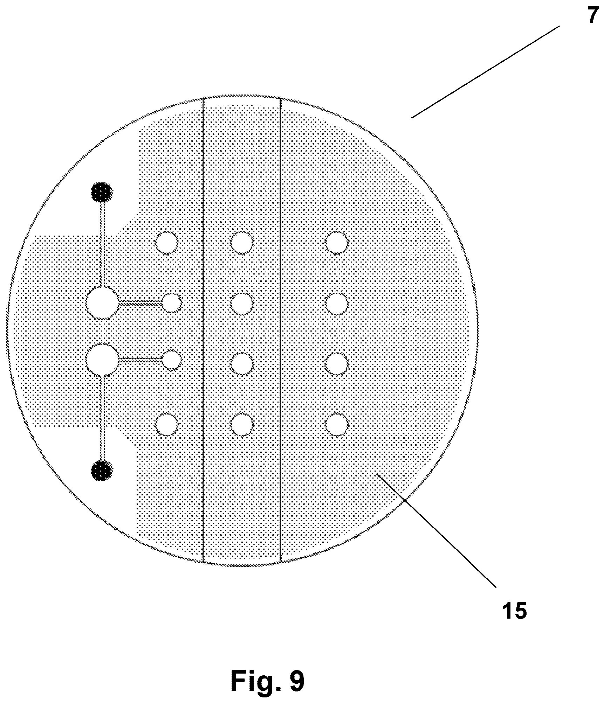

Figure 9 shows the configuration example of Figure 2 with the system of a plate (15) made of a material with high thermal conductivity underneath it to achieve a temperature difference between the first and second drops of each system, in which it can be seen that the plate (15) has cutouts in the zone of the wells where the semen is deposited. This could also be achieved, instead of by means of cutouts in the plate, by interposing a thermally non-conductive element between the plate and the zone of the dish where the wells in which the semen is deposited are located.

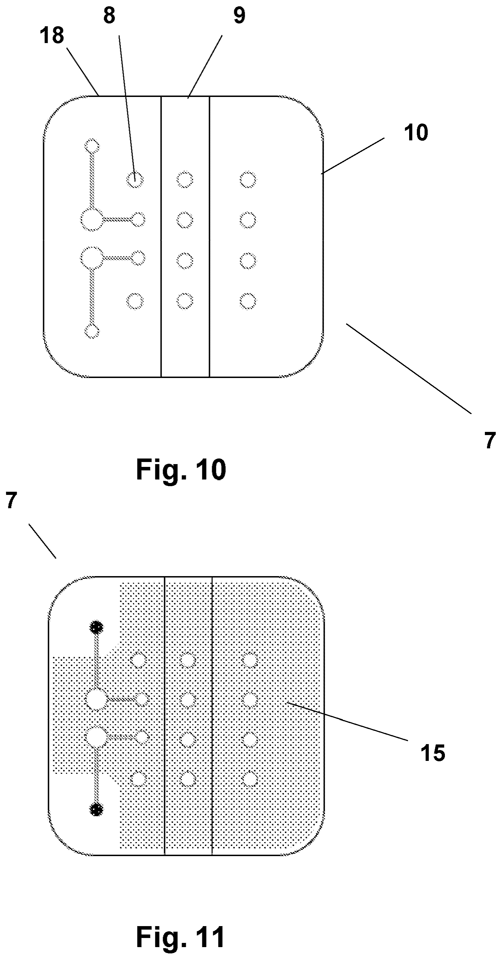

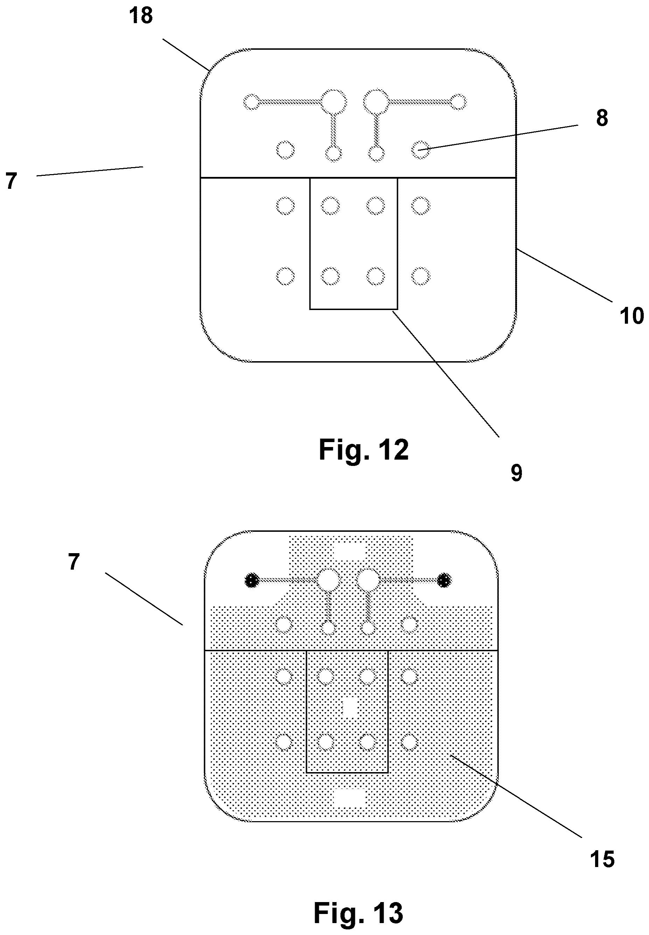

Figures 10 to 13 show examples of rectangular ICSI dishes (7) containing: engraved microfluidic circuits (6) with zones (8) for optionally placing drops of polyvinylpyrrolidone (PVP) or another dense medium where the spermatozoa aspirated from the recovery zones are placed to be immobilized and subsequently microinjected; a washing zone (10) with zones for placing drops of culture medium in which the oocytes can be progressively washed; a microinjection zone (9) with zones for placing drops of culture medium in which the oocytes will be placed for microinjection. The arrangement of the plates made of a material with high thermal conductivity (15) in these ICSI dishes (7) is also shown.

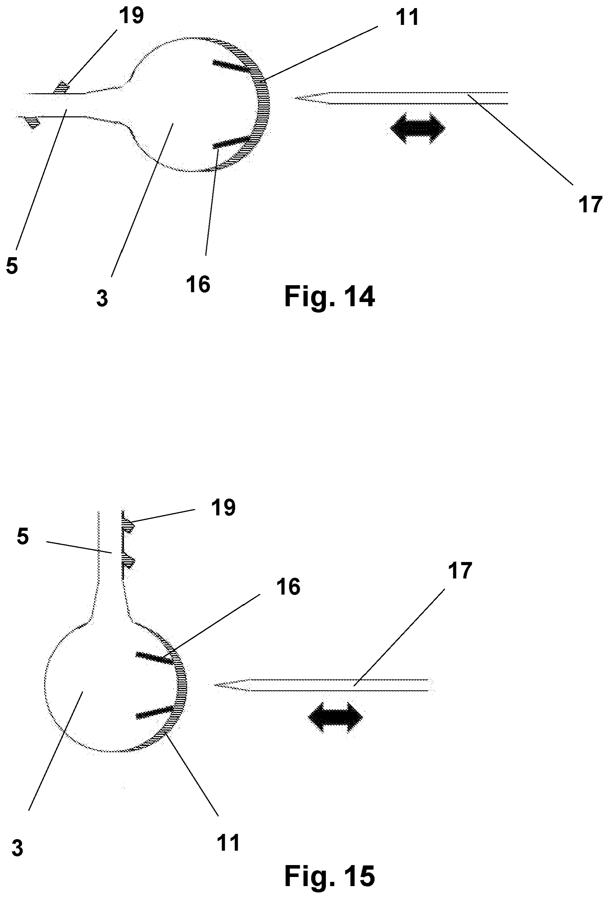

Figures 14 and 15 show examples of different arrangements of the sperm accumulation and recovery zones (11) of the third well (3), and also the sperm recovery zones (19) of the second channel (5). There are also pillars (16) in the third well (3) for redirecting the sperm toward the recovery zone (11).

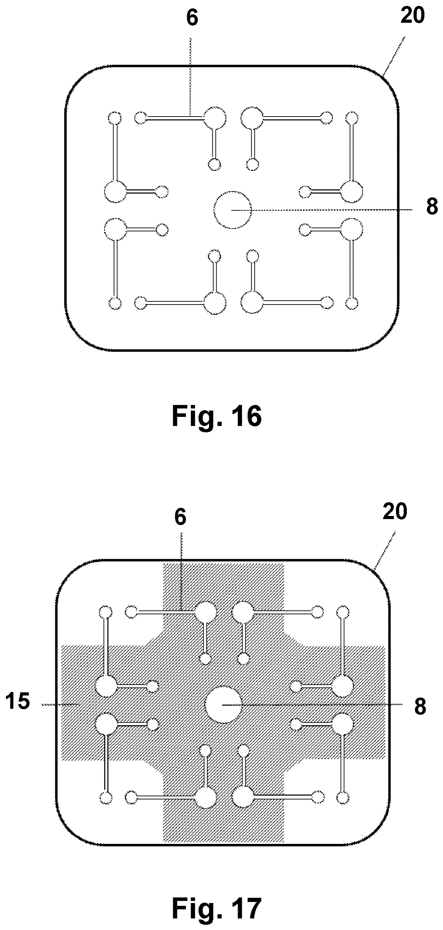

Figures 16 and 17 show an alternative embodiment of the invention in which the dish in which the microfluidic circuits (6) of the sperm selection system are engraved is a dish (20) independent of an ICSI dish, comprising a set of engraved microfluidic circuits (6) and optionally a zone (8) for placing the recovered sperm.

## Claims

1. A sperm selection system, **characterized in that** it comprises a dish with at least one engraved microfluidic circuit (6), in which each microfluidic circuit (6) is formed by three wells (1, 2, 3) and two channels (4, 5); in which the two channels form a capital (L), the length of the first channel (4) being greater than the length of the second channel (5); in which the first well (1) is the well in which the semen sample is deposited and is located at the free end of the first channel (4); in which the third well (3) is located at the free end of the second channel (5); in which the second well (2) is located at the intersection between the first channel (4) and the second channel (5), and has a diameter greater than that of the first well (1) and the third well (3), the first channel (4) corresponding to a dispersion and thermotactic sector and the second channel (5) to a rheotactic sperm selection sector.

2. The sperm selection system as claimed in claim 1, **characterized in that** the depth of the three wells (1, 2, 3) and the two channels (4, 5) is the same.

3. The sperm selection system as claimed in claim 2, **characterized in that** the depth of the three wells (1, 2, 3) and the two channels (4, 5) is 0.1 mm.

4. The sperm selection system as claimed in claim 1, **characterized in that** the first channel (4) has a progressive slope so that it is deeper at the start (at the end in contact with the first well (1)) and shallower at the end (at the end in contact with the second well (2)).

5. The sperm selection system as claimed in any one of claims 1 to 4, **characterized in that** the first channel (4) has a progressive widening in its final section, near the end in contact with the second well (2).

6. The sperm selection system as claimed in claim 4, **characterized in that** the first channel (4) has a section of increasing width (13) in the last 2 mm of its length near the second well (2), its width increasing from 0.5-0.7 mm to 1 mm.

7. The sperm selection system as claimed in any one of claims 1 to 6, **characterized in that** the second channel (5) has a progressive narrowing at its start, so that it is wider at its end in contact with the second well (2).

8. The sperm selection system as claimed in claim 7, **characterized in that** the second channel (5) has a section of decreasing width (13) in the first 2 mm of its length near the second well (2), decreasing in width from 1 mm to 0.5-0.7 mm.

9. The sperm selection system as claimed in any one of claims 1 to 8, **characterized in that** the intersections between the wells and the channels are curved.

10. The sperm selection system as claimed in any one of claims 1 to 9, **characterized in that** the radius of the first well (1) and the third well (3) is the same.

11. The sperm selection system as claimed in claim 10, **characterized in that** the diameter of the first well (1) and the third well (3) is 3 mm.

12. The sperm selection system as claimed in claim 11, **characterized in that** the radius of the second well (2) is 4 mm.

13. The sperm selection system as claimed in any one of claims 1 to 12, **characterized in that** the length of the first channel (4) is 10 mm.

14. The sperm selection system as claimed in any one of claims 1 to 13, **characterized in that** the length of the second channel (5) is 5 mm.

15. The sperm selection system as claimed in any one of claims 1 to 14, **characterized in that** the dish is preferably a polystyrene dish.

16. The sperm selection system as claimed in any one of claims 1 to 15, **characterized in that** the dish comprises more than one engraved microfluidic circuit (6).

17. The sperm selection system as claimed in any one of claims 1 to 10, **characterized in that** the third well (3) has sperm recovery zones (11) formed by progressively shallower protruding parts that cause the sperm to enter and remain trapped therein, ready to be aspirated and used for ICSI.

18. The sperm selection system as claimed in claim 17, **characterized in that** the sperm accumulation and recovery zone (11) has a progressively shallower depth, from 0.1 mm to 1 mm over a distance of 1 mm.

19. The sperm selection system as claimed in either of claims 17 and 18, **characterized in that** there are pillars (16) in the third well (3) for redirecting the sperm toward the recovery zone (11).

20. The sperm selection system as claimed in any one of claims 1 to 19, **characterized in that** along the second channel there is a set of sperm recovery zones (19) formed by progressively shallower protruding parts, arranged on one side of the channel or on both sides.

21. The sperm selection system as claimed in any one of claims 1 to 20, **characterized in that** it comprises a plate (15) made of a material with high thermal conductivity that can be placed or removed from under the dish and that has a specific configuration with a series of cutouts, so that said plate remains in contact with the entire dish except for the wells where the semen sample will be placed.

22. The sperm selection system as claimed in claim 21, **characterized in that** the plate (15) is made of aluminum.

23. The sperm selection system as claimed in any one of claims 1 to 22, **characterized in that** it comprises a cover.

24. The sperm selection system as claimed in any one of claims 1 to 23, **characterized in that** the dish is an ICSI dish (7).

25. The sperm selection system as claimed in claim 24, **characterized in that** the ICSI dish (7) comprises a sperm selection zone (18), which has engraved microfluidic circuits (6) and zones (8) where the sperm aspirated from the recovery zones are placed to be immobilized and subsequently microinjected; a washing zone (10) with zones for placing drops of culture medium where the oocytes can be progressively washed; and a microinjection zone (9) with zones for placing drops of culture medium in which the oocytes will be placed for microinjection.

26. The sperm selection system as claimed in any one of claims 1 to 23, **characterized in that** the dish is an independent dish (20).

27. The sperm selection system as claimed in claim 26, **characterized in that** the independent dish (20) comprises a zone (8) for placing the recovered sperm.

28. The sperm selection system as claimed in any one of claims 1 to 27, **characterized in that** the cross section of the channels (4, 5) of the circuit (6) is rounded.

29. A sperm selection method using the sperm selection system as claimed in any one of claims 1 to 28, **characterized in that** it involves the following steps:
• placing a drop of the same volume of culture medium in each well (1, 2, 3) and then a fourth drop of the same volume on the drop in the second well (2), so that it will have twice the volume of the others;
• passing a sterile pipette tip over the channels, from one well to another, so that the drops become connected if they do not do so by capillarity;
• covering the entire dish with mineral oil;
• placing a drop of semen in the first well (1);
• placing a cover on the dish;
• placing the dish on a plate made of a material with high thermal conductivity (15) that has cutouts so that it is in contact with the entire dish except for the well containing the semen sample;
• placing the dish-plate assembly on a heated flat surface in which the plate made of a material with high thermal conductivity will transmit the temperature to the entire dish except for the well containing the semen sample, creating a temperature gradient between the first well and the second well;
• aspirating sperm from the sperm recovery zones.

30. The sperm selection method as claimed in claim 29, **characterized in that** it is used when the dish is an ICSI dish (7), and comprises a third step consisting in configuring the rest of the ICSI dish by placing thereon the drops that will contain the selected sperm and the oocytes (eggs).
